# EUROPEAN PATENT APPLICATION

(11) **EP 4 692 792 A1**
(43) Date of publication of application: **11.02.2026**
(21) Application number: 24780447.9
(22) Date of filing: 27.03.2024
(51) Int. Cl.: G01N 33/48, G01N 1/10

(54) **SOLUTION AND METHOD FOR TRANSPORTING OR STORING BODY FLUID SAMPLE**

(30) Priority: 31.03.2023 JP 2023057073
(71) Applicant: Provigate Inc., Tokyo 113-0033 (JP)
(72) Inventor: NISHI, Mitsumi, Tokyo 113-0033 (JP); MIYAUCHI, Noriko, Tokyo 113-0033 (JP); SHIMAMURA, Rieko, Tokyo 113-0033 (JP)
(74) Representative: Michalski Hüttermann & Partner Patentanwälte mbB
(86) International application number: PCT/JP2024/012245
(87) International publication number: WO 2024/204342

(57) **Abstract**

An aspect of the present disclosure provides a solution for transporting or storing a body fluid sample, the solution including a substance selected from the group consisting of kanamycin, G418, streptomycin, maltotriose, N-acetylmuramic acid, and glucose as an active ingredient.

## Description

### TECHNICAL FIELD

The present disclosure relates to a solution and a method for transporting or storing a body fluid sample.

### BACKGROUND ART

In blood testing, various biomarkers contained in blood are measured by various methods. One example thereof is glycoalbumin in the blood. Glycoalbumin is a glycated product of albumin. Albumin is a protein that is abundantly present in the body and that has a high glucose-binding rate. The glycation degree (GA value or GA%) of glycoalbumin (GA) is said to represent the average blood glucose level for approximately the past two weeks. Thus, in the recent years, the GA value has drawn much attention as the indicator used in controlling and managing the blood glucose.

The GA value is measured more frequently than HbA1c. Thus, one effective approach is to conduct transport testing rather than the testing in the hospital. Specifically, the user himself/herself collects a body fluid, for example, a minute amount of blood such as fingertip blood or saliva, and sends the body fluid to a testing facility via mail or the like. The testing facility can have measuring devices that can conduct testing at high efficiency and high accuracy, and can provide test results to the user or the related institutions.

However, in a body fluid that is taken outside the body and is being transported or stored, glucose and other components react with proteins such as albumin, and the proteins may become degraded. There are various factors that can change the glycation degree. Thus, the passage of time could affect the final and actual outcome of the glycated protein assay.

### SUMMARY OF INVENTION

Actually, the inventors have found that albumin reacts with glucose in the blood and becomes glycated during transport or storage, and that the assay values can change.

Specifically, the inventors have observed the increases and the decreases in glycation degree. The increase in glycation degree is considered to be proportional to the frequency of physical contact between albumin and glucose. The inventors have found that increasing the dilution factor can reduce the increase in glycation degree and thus can substantially eliminate the influence on the assay values. Meanwhile, the decrease in glycation degree is considered to be caused by unbinding of glucose from the protein.

### <Examples of the decrease in glycation degree>

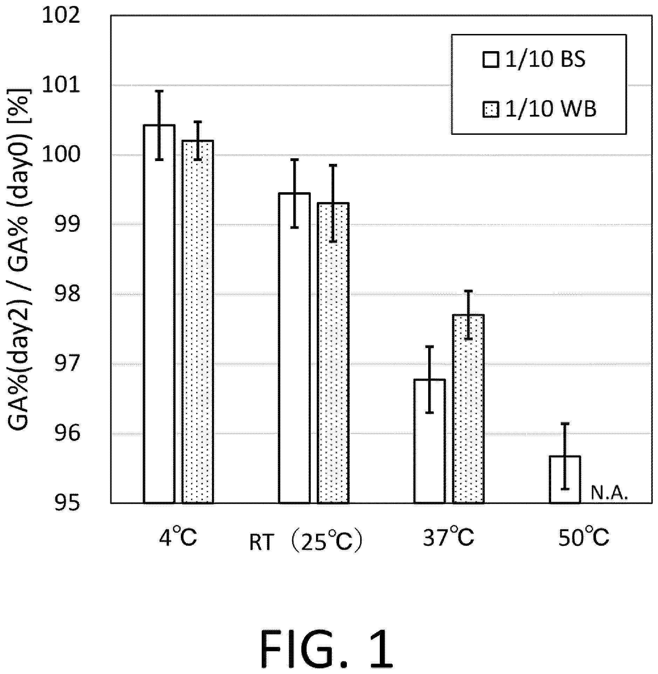

Fig. 1 indicates the influence of the temperature on the GA value of glycoalbumin as one example. Blood serum (BS) and whole blood (WB) were used as blood samples. Pool serum was diluted 20-fold to prepare a serum sample. Pool whole blood was diluted 10-fold to prepare a whole blood sample. Diluting can reduce the increase in GA value over time. Samples immediately after dilution ("day 0") and samples left standing still for two days at 4°C, at room temperature (25°C), and in a constant temperature reservoir (37°C and 50°C) ("day 2") were prepared. HPLC was employed to assay the GA value of each sample. The graph in Fig. 1 assumes the GA value of day 0 as 100 and indicates the ratios (relative GA values) of the GA values of day 2 with respect to this day 0.

The relative GA value did not change substantially at 4°C (about 100%). For both the blood serum (BS) and the whole blood (WB), the relative GA values decreased from 25°C and decreased as the temperature increased to 37°C and 50°C. For the whole blood (WB), the relative GA value was below 98% at 37°C. The relative GA value of the whole blood left standing still at 50°C for two days was not measured ("N.A." in the graph). For the blood serum (BS), the relative GA value was below 97% at 37°C and below 96% at 50°C.

It was found from at least the relative GA value at 37°C that the GA value of the blood serum was more prone to decrease by heat than the GA value of the whole blood. The cause for this difference between the blood serum and the whole blood is not clear. However, one hypothesis is the influence of the blood cell components. In other words, the GA value possibly increased due to some contaminants generated by hemolysis.

The influence of the high-temperature environment on the GA value can pose a problem in transport testing. In many cases, the temperature in the mail cannot be controlled. Meanwhile, the region where the mail testing is conducted may have a weather that can bring a high temperature. Moreover, the blood sample may be stored in a sealed container that can be exposed to sunlight. In such a case, the temperature of the blood sample can rise. If mailing takes few days, the blood sample may be exposed to a high-temperature environment. As a result, a correct GA value cannot be sent back to the subject. Even if this issue can be controlled by refrigerated transport, the testing businesses must accept the high cost. A technique that enables transport testing of the GA value at low cost, for example, without temperature control, is desirable.

One object of the present disclosure is to practically eliminate or reduce changes in the glycation degree of proteins contained in a body fluid sample even when the body fluid sample is exposed to a high-temperature environment during storage or mailing.

In some embodiments of the present disclosure, a solution for transporting or storing a body fluid sample is provided. In some embodiments, the body fluid sample may be a blood sample or a saliva sample. In some embodiments, the solution may contain a substance selected from the group consisting of kanamycin, G418, streptomycin, maltotriose, and N-acetylmuramic acid as an active ingredient.

Further aspects and advantages of the present disclosure will become easily apparent to a person skilled in the art from the following detailed description in which only the illustrative embodiments of the present disclosure will be described. Obviously, the present disclosure can encompass various other embodiments, and some of the details thereof can be altered through various obvious features without departing from the scope of the present disclosure. Thus, the drawings and the description should be deemed merely exemplary and not limiting.

### BRIEF DESCRIPTION OF DRAWINGS

[Fig. 1] Fig. 1 is a graph indicating relative GA values after two days of standing at respective temperatures according to one example.
[Fig. 2] Fig. 2 is a graph indicating relative GA values when each active ingredient was used according to one example.
[Fig. 3] Fig. 3 is a graph indicating relative GA values when each active ingredient was used according to one example.
[Fig. 4] Fig. 4 is a graph indicating relative GA values when each active ingredient was used according to one example.
[Fig. 5] Fig. 5 is a graph indicating relative GA values when each active ingredient was used according to one example.

### <Body fluid>

The term "body fluid sample" as used herein generally refers to a body fluid of an animal directly, a liquid derived from a body fluid, or a liquid that is not a body fluid but is prepared in relation to a body fluid (non-body-fluid-derived liquid). A body fluid sample may be a diluted liquid of a body fluid. The body fluid sample may be a solution other than a body fluid (non-body-fluid-derived solution) or a mixture of a body fluid or a body-fluid-derived liquid, and a non-body-fluid derived liquid. A body fluid sample may be a fractionated liquid or cultured liquid of micro substances, cells, extracellular vesicles, etc. A body fluid sample may be a liquid used in sample assay or a liquid used in calibration assay. For example, a body fluid sample may be a standard liquid or a calibration liquid. For example, a body fluid sample may be a liquid intentionally or systematically free of the assay target substance for the purposes of use in calibration or the like. The sample to be assayed may be a specimen. A body fluid sample may be a liquid containing a chemical substance.

A "body fluid" may be what is generally known as an extracellular fluid. Examples of the body fluid include, but are not limited to, blood; lymph; tissue fluid (such as interstitial fluid, intercellular fluid, and interstitial fluid); and body cavity fluid (such as serous cavity fluid, pleural effusion, peritoneal effusion, pericardial fluid, cerebrospinal fluid (spinal fluid), joint fluid (synovial fluid), and fluid in the chambers of the eye (aqueous humor)). The body fluid may be digestive fluid such as saliva, gastric juice, bile, pancreatic juice, or intestinal juice, or may be sweat, tears, nasal mucus, urine, semen, vaginal fluid, amnion fluid, or milk.

In some embodiments, the body fluid may be a human body fluid.

The "body fluid" may be a solution. The solution may contain a biological buffer, such as phosphate buffered saline (PBS) or N-tris(hydroxymethyl)methyl-2-aminoethanesulfonic acid buffer (TES), that contains a target substance. The solution is not particularly limited as long as the target substance is contained.

A body fluid sample may contain a target substance. A body fluid sample may have a possibility of containing a target substance or may be suspected to contain a target substance. In some embodiments, the target substance may be a molecule, an ion, a polymer, a biomolecule, or the like. The target substance may include a biomolecule. The target substance may be a biological material. The target substance may be a protein, a glycated protein, or the like. The target substance may be a substance related to glycation. For example, the target substance may be a protein to be glycated or a glycated protein with which the glycation degree decreases by the presence of glucose. The target substance may be albumin, glycoalbumin, hemoglobin, and/or glycohemoglobin in blood, blood serum, or blood plasma. For example, the target sample may be albumin and/or glycoalbumin in interstitial fluid, urine, or saliva. For example, the body fluid sample may be tears and the target substance may be albumin and/or glycoalbumin contained in the tears. Albumin may be oxidized albumin (HNA) or reduced albumin (HMA). In some embodiments, the target substance may be advanced glycation end products (AGEs). In some embodiments, the target substance may be a glycated lipid.

### <Blood>

A blood sample can be collected by various techniques. In some embodiments, the blood sample may be capillary blood. In some embodiments, the blood sample may be taken from a vein or an artery of a living organism. In some embodiments, the blood sample may be collected by puncture. The blood sample may be collected from a droplet (capillary blood) on a skin formed by puncturing a fingertip, an earlobe, a sole, an arm, an abdomen, or the like. In some embodiments, the blood sample may be taken by the subject himself/herself. In some embodiments, the blood sample may be taken by a doctor, a nurse, or a clinical laboratory technician. In some embodiments, the blood sample may be taken near a testing device (for example, in a clinic where the testing device is installed).

In some embodiments, the amount of the blood sample taken from the subject may be a micro amount. A micro amount of blood may have a volume of a droplet (capillary blood) of a micro amount of blood formed on skin by puncture.

The volume of fingertip blood is generally in the range of 0.5 µL to 20 µL. In some embodiments, the volume of the fingertip blood is 10 µL. A predetermined volume of blood may be collected.

The volume may be equal to or larger than a value such as 0.1 µL, 0.2 µL, 0.3 µL, 0.4 µL, 0.5 µL, 0.6 µL, 0.7 µL, 0.8 ilL, 0.9 µL, 1.0 µL, 1.1 µL, 1.2 µL, 1.3 µL, 1.4 µL, 1.5 ilL, 1.6 µL, 1.7 ilL, 1.8 µL, 1.9 ilL, or 2.0 µL.

The volume may be equal to or smaller than a value such as 1.0 µL, 1.5 µL, 2 µL, 3 µL, 4 µL, 5 µL, 6 µL, 7 ilL, 8 µL, 9 µL, 10 µL, or 15 µL.

In some embodiments, the blood sample to be mixed with a diluent may be whole blood. In some embodiments, the blood sample to be mixed with a diluent may be blood plasma or blood serum.

### <Saliva>

In some embodiments, the body fluid sample may be saliva. Saliva can be collected by various methods. For example, the subject may spit saliva secreted naturally in the mouth or by stimuli into a cup (passive drool method or PD). For example, an absorber such as cotton may be placed in the mouth and allowed to absorb saliva. For example, saliva in the mouth may be suctioned by a dropper. For example, saliva may be guided from the mouth into a tube by using a funnel or a straw. For example, a mouthwash liquid may be put in the mouth and a mixture of the mouthwash liquid and saliva may be guided into a tube.

### <Solution>

The term "solution" as used herein refers to a solution that is mixed with a body fluid sample to dilute the body fluid sample unless otherwise noted, and is a term that is used interchangeably with a "diluent".

In some embodiments, the diluent may contain a substance (active ingredient) that has an ability to reduce changes overtime in glycation degree of proteins substantially caused by glycation. In some embodiments, the active ingredient may be selected from the group consisting of kanamycin (C₁₈H₃₆N₄O₁₁), G418 (C₂₀H₄₀N₄O₁₉), streptomycin (C₂₁H₃₉N₇O₁₂), maltotriose (C₁₈H₃₂O₁₆), N-acetylmuramic acid (C₁₁H₁₉NO₈), and glucose (C₆H₁₂O₆). In some embodiments, the active ingredient may contain one active ingredient. In some embodiments, the active ingredient may contain multiple active ingredients. In some embodiments, the active ingredient may be a salt. Examples of the salt include, but are not limited to, sulfate salts and hydrochlorides. Examples of the active ingredient include, but are not limited to, kanamycin monosulfate, G418 disulfate, and streptomycin sulfate.

The concentration of the active ingredient in the solution may be set such that the assay value of the target glycated protein is not significantly affected from the time the blood is collected or mixed with a solution to the time of the assay. For example:
The concentration of kanamycin may be set to 0.1% or more.

The concentration of G418 may be set to 0.1% to 0.5%.

The concentration of streptomycin may be set to 0.1% or less.

The concentration of maltotriose may be set to 0.2% or less.

The concentration of N-acetylmuramic acid may be set to 0.3% or less.

These have been confirmed to be effective if the time to the assay is within 2 days (see Examples below).

### <Dilution factor>

The phrase "dilution factor" or "N×" as used herein means (V0 + V1)/V0 where V0 is the amount of the body fluid sample taken and V1 is the amount of the diluent solution used. For example, when 1 µL of a collected body fluid is mixed with 9 µL of a diluent solution to prepare 10 µL of a transport liquid or a storage liquid, the dilution factor is "10-fold" or "10×".

It is effective to dilute the body fluid sample to address the increase in glycation degree. The increase in glycation degree is considered to be caused by physical contact between albumin and glucose. Dilution decreases this frequency and contributes to reducing the speed of glycation. Due to the actions of dilution and the active ingredient, a GA value with required accuracy can be obtained so long as the time (for example, several hours, two days, several days, or one week) and the temperature (for example, room temperature or normal temperature) are within realistic ranges. In some embodiments, such sufficient dilution factors can be employed. At an excessively low dilution factor, that is, when an insufficient dilution factor is employed, the GA value changes during transport and the GA value of the subject cannot be accurately determined.

The dilution factor may be equal to or larger than a value such as 5×, 6×, 7×, 8×, 9x, or 10×_{.} In some embodiments, the dilution factor may be 5× or more. In some embodiments, the dilution factor may be more than 8×. In some embodiments, the dilution factor may be 10× or more.

At an excessively high dilution factor, that is, when the concentration of the target protein (for example, albumin) is excessively low, the assay is no longer possible. At least the assay technique and the assay device have the concentration limit. The albumin concentration in the mixture needs to be larger than the limit. The amount of blood collected may be small depending on the subject. The concentration must be in the range in which at least the assay is possible theoretically or practically.

The dilution factor may be equal to or smaller than a value such as 10×, 15×, 20×, 25×, 30×, 40×, 50×, 60×, 70×, 80×, 90×, or 100×. In some embodiments, the dilution factor may be 100× or less. In some embodiments, the dilution factor may be 90× or less. In some embodiments, the dilution factor may be 80× or less. In some embodiments, the dilution factor may be 50× or less. In some embodiments, the dilution factor may be 20× or less.

In some embodiments, the dilution factor may be between 5× and 100×. In some embodiments, the dilution factor may be between 8× and 100×. In some embodiments, the dilution factor may be between 9× and 100×. In some embodiments, the dilution factor may be between 10× and 100×.

### <Chelating agent>

In some embodiments, the solution may contain a chelating agent. In some embodiments, the solution may contain a substance having an ability of capturing divalent cations. It is known that divalent cations such as calcium inhibit binding between albumin and kanamycin (aminoglycoside-based antibiotic). Adding a divalent cation is considered to weaken the action of kanamycin on the decrease in glycation degree by inhibiting binding between kanamycin and albumin. In some embodiments, the chelating agent may be EDTA. Examples of the chelating agent include, but are not limited to, EDTA, EDTA-2Na, EDTA-4Na, EDTA-2K, EGTA (GEDTA), EGTA-AM, BAPTA, BAPTA-4Na, HEDTA, HEDTA-3Na, NTA, NTA-2Na, NTA-3Na, CyDTA, BAPTA, BAPTA-4Na, BAPTA-AM, DTPA, DTPA-3Na, DTPA-5Na, TTHA, TTHA-6Na, HIDS, HIDS-3Na, EDDS, EDDS-3Na, DOTA, HEDP, phytic acid, and cyclen.

### <Buffer>

In some embodiments, the solution may contain a buffer. In some embodiments, the buffer may be selected from the group consisting of HEPES (4-(2-hydroxyethyl)-1-piperazineethanesulfonic acid), sodium acetate, and phosphate buffered saline (PBS).

### <Salt>

In some embodiments, the solution may contain a salt. The salt may be NaCl, KCl, or the like without any limitation. In some embodiments, the salt may be NaCl.

In some embodiments, the solution may be adjusted so as to be an isotonic solution with respect to the blood cell components. As a result, hemolysis can be reduced. Hemolysis generally does not affect the HPLC assay. However, when components such as hemoglobin that has emerged due to hemolysis become denatured or modified due to heat or passage of time, such components may affect the HPLC assay. Furthermore, hemolysis affects optical measurement such as absorbance measurement. Thus, reducing the hemolysis during transport or storage lowers the risk of the assay becoming impossible or inaccurate.

### <GA assay>

The present disclosure does not pose any limit on the GA assay technique. In some embodiments, the GA value may be assayed by HPLC. In some embodiments, the GA value may be assayed by an enzyme method.

An absorbance measurement device that uses an enzyme method generally costs less and does not require a large installation space. Furthermore, commercially available reagents can be used, and condition setting, reagent preparation, etc., are not necessary. Furthermore, the pretreatment operation process is simple. A centrifuged blood collection tube may be directly loaded. However, according to these, accurate assay values cannot be obtained on the basis of the optical measurement such as absorbance measurement when a pigmented substance, such as hemoglobin, is contained, for example, when whole blood is to be assayed. In other words, accurate assay cannot be performed in the case of hemolysis.

In general, an HPLC device can carry out relatively accurate assay in the case of hemolysis also. Since such a device is a dedicated device, the cost per single unit is high, a large installation space (footprint) is necessary, and the assay time is long. However, multiple HPLC devices may be installed in a facility and can be run under appropriate administration at all times. As a result, in addition to high accuracy, high efficiency and low cost can be achieved as a whole.

### <Other components>

In some embodiments, the solution may contain, as active ingredients, a chelating agent, a buffer, and a salt. In some embodiments, the solution may contain, as an active ingredient, an active ingredient containing a chelating agent, a buffer, and a salt. In some embodiments, the solution may contain a chelating agent, a buffer, a salt, and water. In some embodiments, the solution may essentially consist of a chelating agent, a buffer, a salt, and water.

In some embodiments, the solution may contain components other than a chelating agent, a buffer, and a salt. In some embodiments, the solution is preferably free of albumin and glycoalbumin or components that substantially affect the assay. For example, in carrying out the HPLC assay, some preservatives are preferably not used. For example, ProClin (registered trademark) is preferably not used. CMIT and/or MIT is preferably not used. Meropenem trihydrate is preferably not used. The inventors have found that these components have a tendency to yield GA values that are higher than actual values in at least the HPLC assay (not illustrated).

### <Examples>

Examples will now be described. Blood samples were diluted with diluents containing active ingredients according to the embodiments of the present disclosure, and the GA values thereof were assayed by HPLC on the same day and after two days.

### <Preparation of diluent>

First, a solution containing 10 mM of HEPES and 150 mM of NaCl was prepared. The pH was 8.0. Into this solution, an active ingredient was mixed to prepare a diluent.

### <Preparation of assay samples>

In Examples 1 to 4, pool serum was used as the blood samples. The pool serum was prepared by isolating serum from venous blood taken from multiple healthy subjects and then mixing. The resulting product was mixed with the diluent described above. Into 90 µL of the diluent, 10 µL of the blood serum was introduced to prepare a 10-fold dilution (10×) assay sample.

In Example 5, 1000 mg/dL of glucose was added to the aforementioned diluent. Whole blood of healthy subjects was used as the blood sample. The whole blood sample was introduced into the aforementioned diluent to prepare assay samples of 2×, 10×, and 40× dilution series.

The sample was assayed on the same day ("day 0") or was stored in a 37°C constant temperature reservoir for two days ("day 2") and then assayed.

### <HPLC assay>

For the assay conditions, "GA detection by HPLC method" J Chromatogr 597, 271-275 (1992) was referred. Columns used were AEX:Shodex-Asahipak ES-502N (7.5 mm ID × 100 mm) (Showa Denko K.K.) and BAC:TSKgel Boronate-5PW (4.6 mm ID × 100 mm) (TOSOH CORPORATION). The eluents used were glycine (JIS special grade), magnesium chloride hexahydrate (JIS special grade), D(-)-sorbitol (Wako 1st grade), ethanol (99.5%, JIS special grade), tris(hydroxymethyl)aminomethane (Tris, Biotechnology grade), and EDTA-2Na. For the analysis, Prominence HPLC system (Shimadzu Corporation) equipped with a system controller was used.

### <Descriptions of graphs>

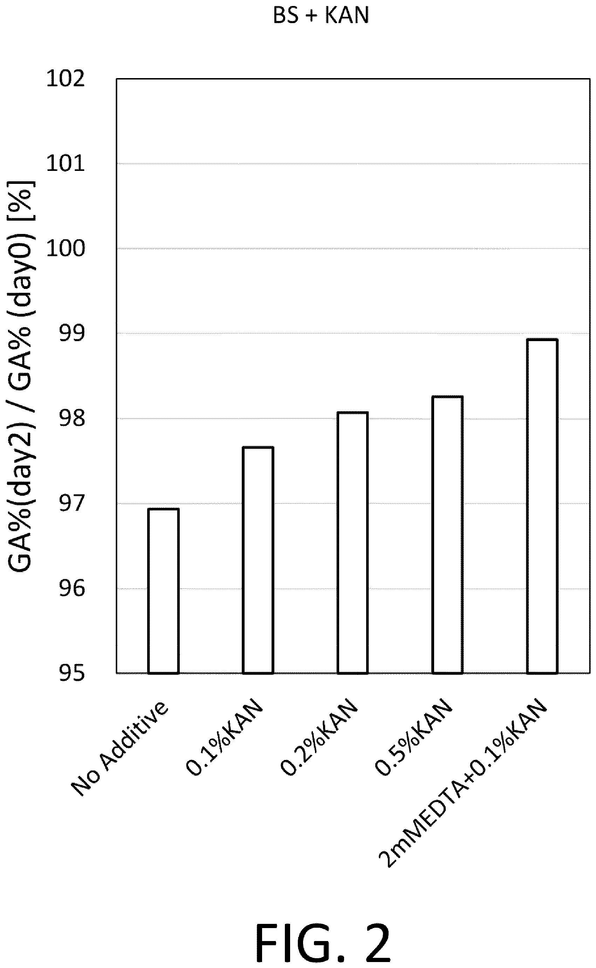

In the graphs indicated in Figs. 2 to 5, the vertical axis indicates the ratio (relative GA value) of the GA value assayed two days (day 2) after the preparation to the GA value measured on the same day (day 0) as the preparation. When assay was carried out multiple times, the average value is indicated.

The horizontal axis indicates the active ingredient group in each of the examples. No additive used (No Additive) is common in all Figs. 2 to 5.

### <Example 2: addition of kanamycin and EDTA>

The following diluent series in which kanamycin was used as an active ingredient were prepared.

### No additive (No Additive);

0.1 w/v% kanamycin (0.1% KAN);
0.2 w/v% kanamycin (0.2% KAN);
0.5 w/v% kanamycin (0.5% KAN); and
0.1 w/v% kanamycin and 2 mM EDTA (2mM EDTA + 0.1% KAN)

Each of the additives was dissolved in the diluent to a predetermined concentration (0.1% to 0.5%) in w/v. For example, specifically, 570 µL of the diluent containing each additive was added to 30 µL of pool serum to prepare a 20× diluted liquid. The GA% of the day 0 sample and that of the day 2 sample left to stand still for 2 days in a 37°C constant temperature reservoir were assayed by using HPLC.

As indicated in Fig. 2, the relative GA value was below 97% when the kanamycin additive was absent (No Additive). In contrast, addition of kanamycin yielded a smaller rate of decrease (0.1% to 0.5% KAN). As the kanamycin concentration increased, the decrease in relative GA value was further reduced.

It was found that the effect thereof is reinforced by further adding EDTA. The relative GA value for "2 mM EDTA + 0.1% KAN" (about 99%) was not only larger than the value for "0.1% KAN" (about 97.6%) but also larger than the value for "0.5% KAN" (about 98.2%). In other words, this indicates the possibility that addition of EDTA could decrease the amount of kanamycin to be added.

### <Example 3: streptomycin and G418>

The following dilution series of streptomycin and G418 were prepared.

### No additive (No Additive);

0.2 w/v% kanamycin (0.2% KAN);
0.1 w/v% streptomycin (0.1% STR);
0.2 w/v% streptomycin (0.2% STR);
0.1 w/v% G418 (0.1% G418); and
0.2 w/v% G418 (0.2% G418).

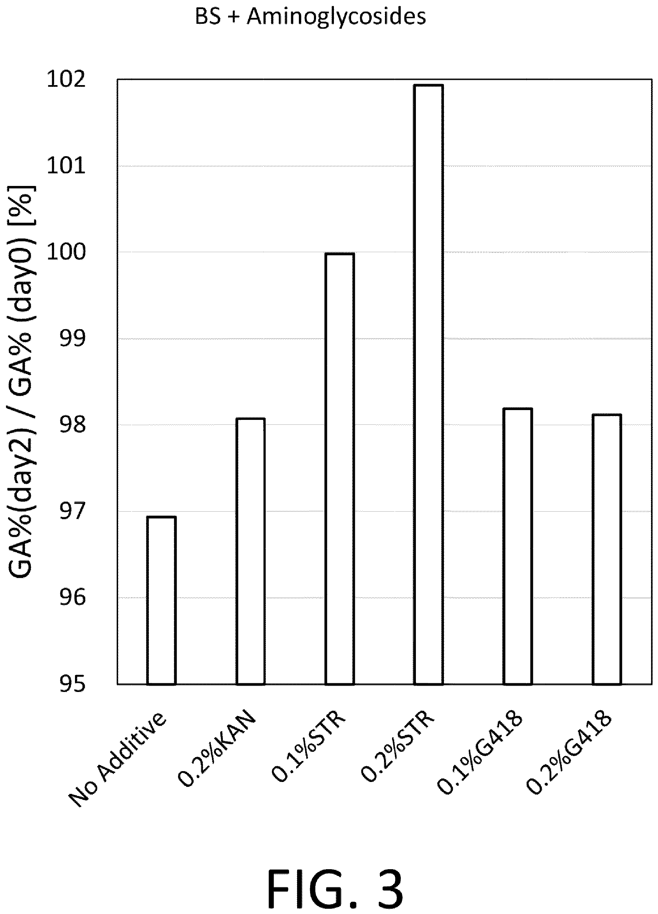

As indicated in Fig. 3, the relative GA value for "0.1% STR" was about 100%. In other words, a sufficient effect of reducing the decrease in GA value was exhibited. Meanwhile, the relative GA value for "0.2% STR" was about 102%. This excessively increased the GA value. Thus, it was found that excessive addition of STR produced an adverse effect.

The relative GA value for "0.1% G418" was about 98.2, and this was an effect of reducing the decrease in GA value about the same level as that for "0.2% KAN". The relative GA value for "0.2% G418" was about 98.2, and this was an effect about the same as that for "0.1% G418". In other words, it was found that addition of G418 yielded an effect of reducing the decrease in GA value. Meanwhile, the influence of the concentration was not observed at least for 0.1% and 0.2%.

### <Example 4: maltotriose and N-acetylmuramic acid>

The following dilution series of maltotriose and N-acetylmuramic acid were prepared.

### No additive (No Additive);

0.1 w/v% maltotriose (0.1% MT);
0.2 w/v% maltotriose (0.2% MT);
0.5 w/v% maltotriose (0.5% MT);
0.1 w/v% N-acetylmuramic acid (0.1% NAM);
0.2 w/v% N-acetylmuramic acid (0.2% NAM); and
0.5 w/v% N-acetylmuramic acid (0.5% NAM).

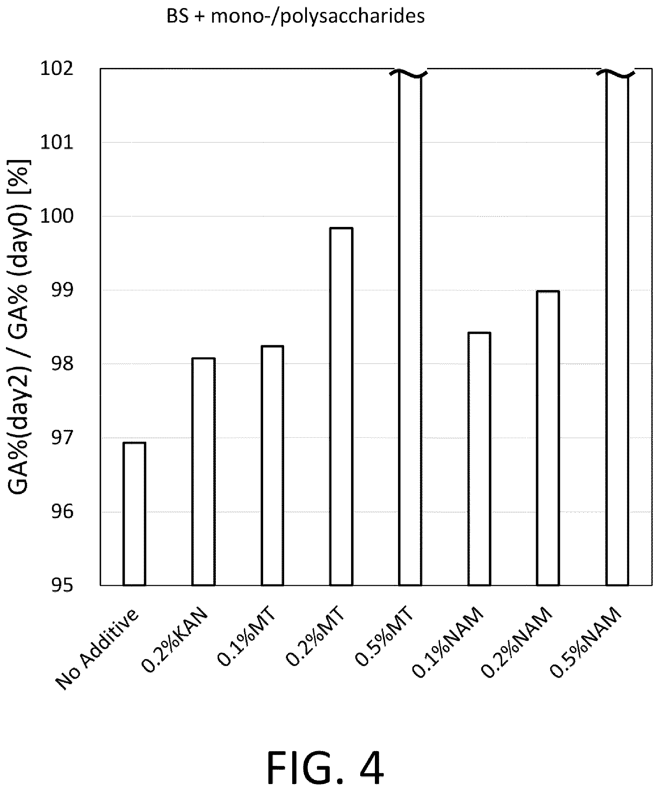

As indicated in Fig. 4, the relative GA value for "0.1% MT" was about 98.2, that is, an effect of reducing the decrease in GA value about the same level as that for "0.2% KAN" was exhibited. The relative GA value for "0.2% MT" was about 99.8, and a sufficient effect of reducing the decrease in GA value was exhibited. Meanwhile, the relative GA value for "0.5% MT" was excessively large (not illustrated). This excessively increased the GA value. Thus, it was found that excessive addition of MT produced an adverse effect.

The relative GA value for "0.1% NAM" was about 98.5, that is, an effect of reducing the decrease in the GA value superior to that for "0.2% KAN" was exhibited. The relative GA value for "0.2% NAM" was about 99.0, and a sufficient effect of reducing the decrease in GA value was exhibited. Meanwhile, the relative GA value for "0.5% MT" was excessively large (not illustrated). This excessively increased the GA value. Thus, it was found that excessive addition of NAM produced an adverse effect.

### <Example 5: glucose>

The following dilution series of glucose were prepared.
diluent with 0.03% glucose added (1/40 (0.03% GLU));
diluent with 0.1% glucose added (1/10 (0.1% GLU)); and
diluent with 0.5% glucose added (1/2 (0.5% GLU)).

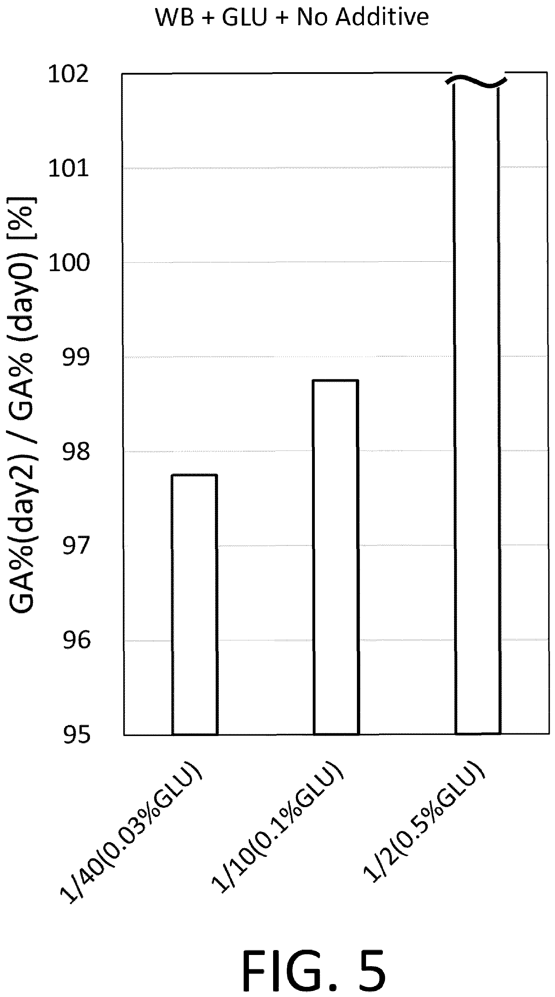

As indicated in Fig. 5, the relative GA value for "1/40 (0.03% GLU)" was about 97.7, and a sufficient effect of reducing the decrease in GA value was exhibited. The relative GA value for "1/10 (0.1% GLU)" was about 98.7, and a superior effect of reducing the decrease in GA value was exhibited. Meanwhile, the relative GA value for "1/2 (0.5% GLU)" was excessively large (not illustrated). This excessively increased the GA value. Thus, it was found that excessive addition of glucose produced an adverse effect.

### <Body fluid storage/transport kit>

In some embodiments, a body fluid storage/transport kit (in the present description, may be simply referred to as a "transport kit") is provided. In some embodiments, a kit may include a solution containing an active ingredient of the present disclosure and a tube that contains the solution. The kit may further include a tool for taking a body fluid. In some embodiments, the tube may contain an active ingredient in a solid state. For example, a liquid may be added to the tube to prepare a predetermined solution containing the active ingredient. A body fluid sample may be introduced into the prepared solution containing the active ingredient. For example, a body fluid sample may be introduced into the tube containing an active ingredient in a solid state. The active ingredient may be mixed with a body fluid sample without dilution. A solid-state active ingredient may be retained, supported, or stored in the tube to be appropriate for storage and/or transport.

In some embodiments, a blood transport kit is provided. In some embodiments, the kit may include a solution containing an active ingredient of the present disclosure and a tube that contains the solution. The kit may further include a blood collecting tube. The blood collecting tube may have a capillary for collecting blood. The kit may also include a plunger that is to be inserted into the blood collecting tube and is configured to push out blood in the capillary. The kit may include a puncture device for blood collection.

In some embodiments, a saliva transport kit is provided. In some embodiments, the kit may include a solution containing an active ingredient of the present disclosure and a tube that contains the solution. Saliva may be directly put in a tube containing the solution. The kit may include cotton, a funnel, a straw, a dropper, or the like for collecting saliva.

The present disclosure includes following embodiments: A001

A solution for transporting or storing a body fluid sample, the solution including:
a substance selected from the group consisting of kanamycin, G418, streptomycin, maltotriose, N-acetylmuramic acid, and glucose as an active ingredient.

### A001b

A solution for transporting or storing a body fluid sample, the solution including:
a substance selected from the group consisting of kanamycin, G418, streptomycin, maltotriose, and N-acetylmuramic acid as an active ingredient.

### A001c

A solution for transporting or storing a body fluid sample, the solution including:
kanamycin as an active ingredient.

### A001d

A solution for transporting or storing a body fluid sample, the solution including:
an active ingredient selected from the group consisting of kanamycin, aminoglycoside, and a mono/polysaccharide, the active ingredient having an ability of reducing a decrease in glycation degree of a glycated protein.

### A001e

A solution for transporting or storing a body fluid sample, the solution including:
an active ingredient having an ability of reducing a decrease in glycation degree of a glycated protein.

### A005

The solution described in one of A001 to A001c or one of the embodiments, in which
a concentration of the kanamycin is 0.1% to 0.3%;
a concentration of the G418 is 0.1% to 0.3%;
a concentration of the streptomycin is 0.1% or less;
a concentration of the maltotriose is 0.1% to 0.2%; and
a concentration of the N-acetylmuramic acid is 0.1% to 0.3%.

### A006

The solution described in one of A001 to A005 or one of the embodiments, in which
the solution further includes a chelating agent.

### A007

A solution for transporting or storing a body fluid sample, the solution including:
kanamycin and a chelating agent as active ingredients.

### A008

The solution described in A007 or one of the embodiments, in which
a concentration of the kanamycin is 0.1% to 0.3%; and
the chelating agent is EDTA.

### A011

The solution described in A001 or one of the embodiments, in which
the body fluid sample is blood, saliva, lacrimal fluid, or a solution derived therefrom.

### A012

The solution described in A001 or one of the embodiments, in which
the body fluid sample is suspected to contain a glycated protein.

### A013

The solution described in A012 or one of the embodiments, in which
the glycated protein is glycoalbumin.

### B001

A substance or solution used for reducing a decrease in glycation degree of a glycated protein, the substance or solution including:
a substance selected from the group consisting of kanamycin, N-acetylmuramic acid, maltotriose, streptomycin, G418, and glucose as an active ingredient.

### B002

The substance or solution described in B001 or one of the embodiments, in which
the substance or solution is used for transporting or storing a body fluid sample.

### B011

The solution described in B001, B002, or one of the embodiments, in which
the body fluid sample is suspected to contain a glycated protein.

### C001

Use of a substance selected from the group consisting of kanamycin, N-acetylmuramic acid, maltotriose, streptomycin, G418, and glucose for reducing a decrease in glycation degree of a glycated protein.

### D001

A method for transporting or storing a body fluid sample, the method including:
providing a body fluid sample suspected to contain a glycated protein;
mixing the target solution with a component that reduces a decrease in glycation degree of the glycated protein; and
transporting or storing the mixed solution.

### D002

The method described in D001 or one of the embodiments, in which
the body fluid sample is a body fluid.

### D003

The method described in D001, D002, or one of the embodiments, in which
the glycated protein is glycoalbumin.

### E001

A kit for transporting a body fluid sample, the kit including:
the solution described in one of A001 to A013 and B001 to B011 or one of the embodiments.

### E011

A kit including:
a) the solution described in one of A001 to A013 and B001 to B011 or one of the embodiments; and
b) a tube containing the solution and configured to receive the body fluid sample.

### E012

The kit described in E011 or one of the embodiments, the kit further including:
c) a capillary configured to collect a predetermined volume of the body fluid sample.

### E012b

The kit described in E011 or one of the embodiments, the kit further including:
c0) a collecting tool for collecting the body fluid sample.

### E013

The kit described in E011 or E012 or one of the embodiments, in which
an amount of the solution is 90 µL, and
a volume of a body fluid that the capillary can collect is 10 µL.

### E014

The kit described in one of E001 to E013 or one of the embodiments, in which
d) a plunger that is to be inserted into the capillary and is configured to push out blood in the capillary.

### E021

The kit described in one of E001 to E013 or one of the embodiments, in which
the body fluid sample is blood.

### E022

The kit described in E021 or one of the embodiments, the kit further including:
e1) a puncture device for blood collection.

### E023

The kit described in E021 or E022 or one of the embodiments, in which
an amount of the solution is 90 µL, and
the blood is puncture blood.

### E031

The kit described in one of E001 to E013 or one of the embodiments, in which
the body fluid sample is saliva.

### E032

The kit described in E031 or one of the embodiments, the kit further including:
e2) at least one of cotton, a funnel, a straw, and a dropper.

In the description above, some of the embodiments and examples of the present disclosure are described; however, these embodiments and examples merely illustrate exemplary examples of the present disclosure. For example, each of the embodiments above is described in detail to facilitate understanding of the present disclosure, and the dimensions, structures, materials, and circuits may be added or changed as necessary. Note that embodiments in which one or multiple features of the present disclosure are combined as desired are also within the scope of the present disclosure. The claims encompass numerous alterations and modifications to the embodiments without departing from the technical spirit of the present disclosure. Thus, the embodiments and examples disclosed in the present description are merely illustrative and should not be interpreted as limiting the scope of the present disclosure.

## Claims

1. A solution for transporting or storing a body fluid sample, the solution comprising:
a substance selected from the group consisting of kanamycin, G418, streptomycin, maltotriose, N-acetylmuramic acid, and glucose as an active ingredient.

2. The solution according to Claim 1, wherein
a concentration of the kanamycin is 0.1% to 0.3%;
a concentration of the G418 is 0.1% to 0.3%;
a concentration of the streptomycin is 0.1% or less;
a concentration of the maltotriose is 0.1% to 0.2%; and
a concentration of the N-acetylmuramic acid is 0.1% to 0.3%.

3. The solution according to Claim 1 or 2,
further comprising a chelating agent.

4. The solution according to Claim 3, wherein
the chelating agent is EDTA.

5. The solution according to Claim 1, wherein
the body fluid sample is blood, saliva, lacrimal fluid, or a solution derived therefrom.

6. The solution according to any one of Claims 1 to 4, wherein
the body fluid sample is suspected to contain glycoalbumin.

7. A method for transporting or storing a body fluid sample, the method comprising:
providing a target solution suspected to contain glycoalbumin;
mixing the target solution with a substance selected from the group consisting of kanamycin, G418, streptomycin, maltotriose, N-acetylmuramic acid, and glucose; and
transporting or storing the mixed solution.

8. A kit for transporting or storing a body fluid sample, the kit comprising:
a) a solution containing a substance selected from the group consisting of kanamycin, G418, streptomycin, maltotriose, N-acetylmuramic acid, and glucose as an active ingredient; and
b) a tube containing the solution and configured to receive the body fluid sample.

9. The kit according to Claim 8, further comprising
c) a collecting tool for collecting the body fluid sample.

10. The kit according to Claim 9, wherein
the body fluid sample is blood, and
the collecting tool includes a capillary.

11. The kit according to Claim 10,
further comprising a puncture device for blood collection.

12. The kit according to Claim 9,
the body fluid sample is saliva, and
the collecting tool includes at least one of cotton, a funnel, a straw, and a dropper.

## Amended claims

### Amended claims under Art. 19.1 PCT

1. A solution for transporting or storing a body fluid sample, the solution comprising:
a substance selected from the group consisting of kanamycin, G418, streptomycin, maltotriose, N-acetylmuramic acid, and glucose as an active ingredient that reduces a decrease in glycation degree of a glycated protein.

2. The solution according to Claim 1, wherein
a concentration of the kanamycin is 0.1% to 0.3%;
a concentration of the G418 is 0.1% to 0.3%;
a concentration of the streptomycin is 0.1% or less;
a concentration of the maltotriose is 0.1% to 0.2%; and
a concentration of the N-acetylmuramic acid is 0.1% to 0.3%.

3. The solution according to Claim 1 or 2,
further comprising a chelating agent.

4. The solution according to Claim 3, wherein
the chelating agent is EDTA.

5. The solution according to Claim 1, wherein
the body fluid sample is blood, saliva, lacrimal fluid, or a solution derived therefrom.

6. The solution according to any one of Claims 1 to 4, wherein
the glycated protein is glycoalbumin.

7. A method for transporting or storing a body fluid sample, the method comprising:
providing a target solution suspected to contain glycoalbumin;
mixing the target solution with a substance selected from the group consisting of kanamycin, G418, streptomycin, maltotriose, N-acetylmuramic acid, and glucose as an active ingredient that reduces a decrease in glycation degree of a glycated protein; and
transporting or storing the mixed solution.

8. A kit for transporting or storing a body fluid sample, the kit comprising:
a) a solution containing a substance selected from the group consisting of kanamycin, G418, streptomycin, maltotriose, N-acetylmuramic acid, and glucose as an active ingredient that reduces a decrease in glycation degree of a glycated protein; and
b) a tube containing the solution and configured to receive the body fluid sample.

9. The kit according to Claim 8, further comprising
c) a collecting tool for collecting the body fluid sample.

10. The kit according to Claim 9, wherein
the body fluid sample is blood, and
the collecting tool includes a capillary.

11. The kit according to Claim 10,
further comprising a puncture device for blood collection.

12. The kit according to Claim 9,
the body fluid sample is saliva, and
the collecting tool includes at least one of cotton, a funnel, a straw, and a dropper.
